Europäisches Patentamt

European Patent Office

Office européen des brevets

(11) Publication number: **0 255 206 B1**

# EUROPEAN PATENT SPECIFICATION

(45) Date of publication of patent specification: **22.03.95** (51) Int. Cl.6: **C07K 7/08**, A61K 38/00

(21) Application number: **87304615.5**

(22) Date of filing: **22.05.87**

(54) **Peptides that inhibit von Willebrand factor binding.**

(30) Priority: **30.05.86 US 869188**

(43) Date of publication of application:
**03.02.88 Bulletin  88/05**

(45) Publication of the grant of the patent:
**22.03.95 Bulletin  95/12**

(84) Designated Contracting States:
**AT BE CH DE ES FR GB GR IT LI LU NL SE**

(56) References cited:
**WO-A-85/04585**

**The Journal of Biological Chemistry, vol. 261, no. 1, January 5, 1986, pages 381-385, Am Soc. of Biol. Chem., Inc., US. Y Fujimura et al.**

**The Journal of Biological Chemistry, vol. 262, no. 4, February 5, 1987, pages 1734-1739, Am. Soc. of Biol.Chem., Inc., US. Y Fujimura et al.**

**J Clin. Invest., vol. 77, March 1986, pages 743-749 Am Soc. for Clin. Inv.Inc., US. P Bockenstedt et al.**

(73) Proprietor: **SCRIPPS CLINIC AND RESEARCH FOUNDATION
10666 North Torrey Pines Road
La Jolla
California 92037-1093 (US)**

(72) Inventor: **Zimmerman, Theodore S.
544 Bonair Street
La Jolla
California (US)**
Inventor: **Fujimura, Yoshihiro
133-1 Sugaharahigashi-cho
Nara City
Nara (JP)**
Inventor: **Houghten, Richard A.
558 Ford Avenue
Solana Beach
California (US)**
Inventor: **Ruggeri, Zaverio M.
644 Bonair Street
La Jolla
California (US)**

(74) Representative: **Read, Matthew Charles et al
Venner Shipley & Co.
20 Little Britain
London EC1A 7DH (GB)**

Proc. Natl. Acad. Sci., USA, vol. 82, October 1985, pages 6394-6398. J Evan Sadler et al.

Biochemistry, vol. 25, December 30, 1986, pages 8537-8361; Am. Chem. Soc., US. G J Roth et al.

**Description**

Background of the Invention

This invention relates to peptides which inhibit the binding of von Willebrand factor (vWF) to platelets, heparin and collagen.

This invention also relates to the prevention of thrombosis.

vWF is known to play a central role in the earliest stages of platelet deposition at the site of vessel wall injury. When the endothelial cell lining of a blood vessel is broken the subendothelium exposed vWF is required for the subsequent adhesion of platelets to the subendothelium. It does this by binding to one or more components of the subendothelium which may include the heparin-like glycosaminoglycans present there. vWF also binds to the platelet glycoprotein (GP) Ib receptor which causes platelets to adhere to the subendothelium. Binding of vWF to the GPIb receptor in turn triggers binding of fibrinogen to the platelet GPIIb/IIIa receptor and subsequent platelet aggregation.

The deposition of platelets at the site of vessel injury or malformation is thought to play an important role in thrombus formation in a number of disease states including coronary artery occlusion and stroke. In addition, it probably contributes to the occlusion of arterial grafts which may occur when either autologous vein segments or woven artificial graft protheses are used. Platelet thrombus formation may also contribute to the thrombosis which can complicate attempts to relieve vessel obstruction by angioplasty.

Therefore, there is a need for a product which prevents platelet deposition at site of vessel injury, whether the injury occurs naturally or is induced as a result of manipulation. The peptides of the present invention have the ability to prevent these undesired platelet depositions.

Fujimara et al., in "von Willebrand factor: A reduced and alkylated 52/48 kDa fragment beginning at amino acid residue 449 contains the domain interacting with platelet glycoprotein Ib", J. Biol. Chem. 261: 381-385 (1986), do not describe the carboxy-terminal sequence which is necessary to fully specify the fragment. Also in this paper, the property of direct binding of the fragment to the platelets was not demonstrated. Fujimara et al., in "A 52/48 kDa tryptic fragment of von Willebrand factor which begins with amino acid residue 449 binds to platelet GP Ib in the absence of ristocetin", Blood 66: 334a (1985), do not fully specify the fragment nor the amino acid sequence responsible for the binding of the fragment to platelets. Sadler et al., in "Cloning and characterization of two cDNAs coding for human von Willebrand factor", Proc. Natl. Acad. Sci. USA 82: 6394-6398 (1985); describe only a partial amino acid sequence of vWF. Sequences thought to be important in heparin binding of thrombin III, histidine rich glycoprotein and apoliprotein E, proteins unrelated in structure and function to vWF were published by Cardin et al., in "Binding of a high reactive heparin to human apoliprotein E: identification of two heparin-binding domains", Biochem. Biophys. Res. Comm. 134: 783-789 (1986), by Koide et al., in "The heparin-binding site(s) of histidine-rich glycoprotein as suggested by sequence homology with antithrombin III", Febs. Lett. 194: 242-244 (1986), by Koide et al., in "the N-terminal sequence of human plasma histidine-rich glycoprotein homologous to antithrombin with high affinity for heparin", Febs. Lett. 141: 222-224 (1982), and by Koide et al., in "Antithrombin III Toyama: replacement of arginine-47 by cysteine in hereditary abnormal antithrombin III that lacks heparin-binding ability", Proc. Natl. Acad. Sci. USA 81: 289-293 (1984). Bockenstedt et al., in "Isolation of a von Willebrand's factor fragment with ristocetin-dependent platelet binding activity using heparin sepharose", Thombos. Haemostas. 54:59 (1985), describe a 295 kDa vWF fragment different from the vWF fragment of the present invention. This paper does not identify those amino acid sequences present in the 295 kDa fragment responsible for binding to heparin and platelets.

Bockenstedt et al., in "Structural basis of a von Willebrand factor binding to platelet glycoprotein Ib and collagen. Effects of disulfide reduction and limited proteolysis of polymeric von Willebrand factor", J. Clin. Invest.. 77: 743-749 (1986), do not use sequence data to describe a vWF fragment different from that of the present invention. This paper shows loss of activity once the fragment after reduction and alkylation loses its tertiary structure. This is in contrast to the present invention where linear sequences of peptides and the reduced and alkylated 52/48 kDa fragment have activity. Sakariassen et al., in "Mediation of platelet adhesion to fibrillar collagen in flowing blood by a proteolytic fragment of human von Willebrand factor", Blood 67: 1515-1518 (1986), also do not use sequence data to describe a vWF fragment different from that of the present invention. Girma et al., in "Mapping of distinct von Willebrand factor domains interacting with platelets GPIb and GPIIb/IIIa and with collagen using monoclonal antibodies", Blood 67: 1356-1366 (1986), describe vWF fragments different from those of the present invention. This paper neither describes the vWF fragments by amino acid sequence or characterizes such fragments by both their amino and carboxy-termini. Also in this paper, the property of direct binding of fragments to the platelets was not demonstrated. Houdijk et al., in "Identity of functional domains on von Willebrand factor by binding of tryptic fragment to collagen and to platelets in the presence of ristocetin", Blood 67: 1498-1503 (1986), do not use sequence data to describe a vWF fragment different from that of the present invention. Also in this paper there is no purification of the vWF fragment and no proof that the vWF fragment blocks intact vWF from collagen or platelets.

According to a first aspect of the present invention there is provided a peptide which inhibits binding of von Willebrand factor to platelets, whose sequence is selected from one of the following sequences or whose sequence is a combination of any of the following sequences:
LCDLAPEAPPPTLPP; PEAPPPTLPPDMAQV; VKYAGSQVASTSEVL; SDPEHCQICHCDVVN; CDVVNLTCEACQEPG; CQEPGGLVVPPSDAP; PSDAPVSPSSLYVED; RIASQVKYAGSQVAS; PSELRRIASQVKYAG; DMMERLRISQKWVRV; EFEVLKAFVVDMMER; KAFVVDMMERLRISQ; HDGSHAYIGLKDRKR; QIFSKIDRPEASRIA; LYVEDISEPPLHDFY; VSPSSLYVEDISEPP; AYIGLKDRKRPSELR; KDRKRPSELRRIASQ; ASRIALLLMASQEPQ; RMSRNFVRYVQGLKK; VTLNPSDPEHCQICH; CQICHCDVVNLTCEA; IPVGIGPHANLKQIR; GPHANLKQIRLIEKQ; SVDELEQQRDEIVSY; EIVSYLCDLAPEAPP; PTLPPDMAQVTVGPG; TVGPGLLGVSTLGPK; LIEKQAPENKAFVLS; and APENKAFVLSSVDEL.

Particularly preferred is a peptide which inhibits binding of von Willebrand factor to platelets, comprising the sequences LCDLAPEAPPPTLPP.

Additionally preferred is a peptide which inhibits binding of von Willebrand factor to platelets, comprising the sequence PEAPPPTLPPDMAQV.

Additionally preferred is a peptide which inhibits binding of von Willebrand factor to platelets, comprising the sequence LYVEDISEPPLHDFY.

Additionally preferred is a peptide whose sequence is a sequential subset of at least five amino acids from any of the sequences set out above and which inhibits binding of von Willebrand factor to platelets.

Additionally preferred is a peptide with one or more R and/or K residues in any combination extending from the amino or carboxy-terminus of a peptide which inhibits binding of von Willebrand factor to platelets.

In a second aspect, the invention further comprises a peptide which inhibits binding of von Willebrand factor to heparin, whose sequence is selected from one of the following sequences or whose sequence is a combination of any of the following sequences:-
KDRKRPSELRRIASQ; LIEKQAPENKAFVLS; SQEPQRMSRNFURYV; KYTLFQIFSKIDRPE; DMMERLRISQKWVRV; LYVEDISEPPLHDFY; ISEPPLHDFYCSRLL; SQEPQRMSRNFVRYV; IPVGIGPHANLKQIR; GPHANLKQIRLIEKQ; TVGPGLLGVSTLGPK; and LKQIRLIEKQAPENK.

Particularly preferred is a peptide which inhibits binding of von Willebrand factor to heparin, comprising the sequence KDRKRPSELRRIASQ.

Additionally preferred is a peptide whose sequence is a sequential subset of at least five amino acids from any of the sequences set out in the foregoing passage, which inhibit binding of von Willebrand factor to heparin.

Additionally preferred is a peptide with one or more R and/or K residues in any combination extending from the amino or carboxy-terminus of a peptide which inhibits binding of von Willebrand factor to heparin.

In a third aspect, the invention involves the use of a polypeptide, whose amino acid sequence is that of a 52/48 KDa fragment of von Willebrand factor or a functionally equivalent homologue, the fragment comprising an animo-terminal sequence beginning with amino-terminal amino acid residue 449 Val and extending no further than amino acid residue 729 Arg, for the manufacture of a medicament for use in a method for inhibiting binding of von Willebrand factor to heparin or collagen in a patient. Preferably the inventive use is for inhibiting thrombosis in a patient.

In a further aspect, the invention involves the use of a therapeutic composition comprising a polypeptide, whose amino acid sequence is that of a 52/48 kDa fragment of von Willebrand factor or a functionally equivalent homologue, the fragment comprising an amino-terminal sequence beginning with amino-terminal amino acid residue 449 Val and extending no further than amino acid residue 729 Arg, in associated with a pharmaceutically acceptable carrier, for the manufacture of a medicament for use in a method for inhibiting binding of von Willebrand factor to heparin or collagen in a patient.

The invention also comprises a therapeutic composition comprising an amount of one or more of the peptides, of the first or second aspects of the invention, effective to inhibit binding of von Willebrand factor to platelets in a patient, in association with a pharmaceutically acceptable carrier.

Polypeptides and peptides of the invention can be useful in a method of inhibiting thrombosis in a patient, which involves administering to the patient an effective amount of one or more of these polypeptides or peptides.

4

Detailed Description of the Invention

As indicated, the present invention encompasses use of a a 52/48 kDa polypeptide fragment which inhibits binding of vWF to heparin and collagen for the manufacture of a medicament. This particular fragment was isolated as tryptic fragment of vWF that migrates in SDS-polyacrylamide gel electrophoresis (SDS-PAGE), under reducing conditions, as a 52/48 kDa doublet. The amino-terminal sequence, VTLNPSDPEHCQ, was found to be identical for both members of the doublet. This amino-terminal sequence was published in Fujimara et al., J. Biol. Chem. 261: 381-385 (1986). The published amino-terminal sequence was further proof that the differences between doublet constituents were only of carbohydrate composition and established the position of this peptide within the intact vWF polypeptide of approximately 2050 amino acid residue as beginning with residue designated 449.

The characterization of the 52/48 kDa fragment by both its amino and carboxy-termini makes possible the identification of the nucleotide sequence that is essential for the expression of the fragment by recombinant DNA techniques. Through the use of such techniques the fragment is produced by bacteria, yeast, or other cells into which the nucleotide sequence for producing the fragment is inserted by techniques known to those of ordinary skill in the art. The identification of only the amino-terminal sequence of the 52/48 kDa fragment in Fujimara et al., J. Biol. Chem. 261: 381-385 (1986) makes it impossible for one of ordinary skill in the art to express the 52/48 KDA fragment since only by knowing the amino and carboxy-termini of a peptide fragment can the nucleotide sequence that encodes for this fragment be isolated.

The carboxy-terminal sequence that is required along with the amino-terminal sequence for recombinant DNA expression of the 52/48 kDa fragment was determined in the following manner. A 55 kDa fragment of vWF which was produced by the same tryptic digestion that produced the 52/48 kDa fragment was found to have NSMVLDVAFVLE as an amino-terminal sequence. This amino-terminal sequence of the 55 kDa fragment was found to be carboxy-terminal to the 52/48 kDa fragment which had already had its amino-terminal sequence determined. Given this information it can be determined that the carboxy-terminus of the 52/48 kDa fragment ends just prior to the amino-terminal sequence of the 55 kDa fragment. Based on the knowledge of the amino-terminal sequence of the 55 kDa fragment and the known published partial amino acid sequence of vWF in Sadler et al., the amino acid sequence of the carboxy-terminal region of the 52/48 kDa fragment was determined. This carboxy-terminal region was determined to extend no further than the amino acid residue designated 729 in the intact vWF polypeptide. For an explanation of the basis for the numbering of the amino acids comprising the intact vWF polypeptide which was used for characterizing the 52/48 kDa fragment, see Fujimara et al., J. Biol. Chem. 261: 381-385 (1986).

With such information a nucleotide sequence can be inserted into the appropriate vector for expression of the 52/48 kDa fragment. For a description of recombinant DNA techniques for cloning vWF fragments, see Ginsburg et al., Science 228: 1401-1406 (1985) and Sadler et al.

Purified vWF for trypsin cleavage was obtained from commercial factor VIII concentrate (Armour Pharmaceutical, Kankakee, IL) by immunoadsorbant chromatography using an anti-vWF monoclonal antibody coupled to Sepharose 4B (Pharmacia) as described in Fulcher and Zimmerman, Proc. Natl. Acad. Sci. USA 79: 1648-1652 (1982). The vWF bound to the antibody was eluted with 3M NaSCN dissolved in 0.01M imidazole, 0.15M NaCl buffer pH 6.8, containing 0.1M L-lysine HCl and 0.02% NaN$_3$. The eluted vWF was dialyzed extensively against 0.05M Tris, 0.15M NaCl, pH 7.35 (TBS) and then concentrated by ultrafiltration with an Amicon PM-30 membrane (Amicon Corp. Danvers, MA) After centrifugation at 10,000xg for 30 minutes at room temperature, the preparation was subjected to HPLC size exclusion chromatography using two BioRad columns (60 x 2.15 cm) mounted in series, one TSK G4000 SW and the other G3000 SW. The columns were equilibrated with 0.2M Na acetate, pH 5.5, containing 5% dimethyl sulfoxide, and run at flow rate of 4 ml/min.

Trypsin digestion was performed in 0.2 M Na acetate buffer, pH 7.0, containing 0.02% NaN$_3$, at 37°C for 2 h, using 2,500 units of trypsin (bovine pancreatic Type I, 15,000 units/mg, Sigma)/mg of vWF. The fragments generated were separated by HPLC size exclusion chromatography in three main fractions (denominated A, B and C). Isolation of the 52/48 kDa doublet mainly contained in fraction B, as well as of other fragments of 13 kDa, 22 kDa, 41 kDa, (all from fraction B) and 55 kDa (from fraction C), was performed by HPLC chromatofocusing, salt gradient elution, and size exclusion chromatography, all in the presence of 6M urea. Reduction and S-carboxy methylation of proteins was achieved by treatment with dithiothreitol, in equal amount (w/w) to protein, for 1h at 37°C, followed by treatment with a 2.7-fold excess (w/w) of iodoacetamide, for 30 min at room temperature(22-25°C) and in the dark.

All samples were finally dialyzed extensively against a buffer composed of 0.05 M Tris, 0.15 M NaCl, pH 7.35, concentrated by ultrafiltration (Amicon Corp., Danvers, MA), and dialyzed again before storage at -70°C. The tryptic fragments of vWF were analyzed by polyacrylamide gel electrophoresis in the presence of sodium dodecyl sulfate (SDS-PAGE) using 5-15% or 10-15% linear gradient gels, under reducing and non-reducing conditions, as recently described. The gels were stained with Coomassie brilliant blue-R (Bio-Rad Laboratories). The NH$_2$-terminal sequence analysis of each of the purified fragments was performed using a gas-phase sequenator (Model 470A, Applied Biosystems, Foster City, CA). Identification of the phenylthiohydantoin amino acid derivatives was achieved by reverse phase chromatography, in an HPLC system (Perkin-Elmer, Norwalk, CT), using a Zorbax PTH column (E.I. du Pont de Nemours) following the manufacturer's instructions.

Peptides 15 amino acid residues in length beginning from the carboxy-terminal region of the 52/48 fragment were synthesized as described by Houghton et al., in "General method for the rapid solid-phase synthesis of large numbers of peptides: Specificity of antigen-antibody interaction at the level of individual amino acids", Proc. Natl. Acad. Sci. 82:5131-5135 (1985).

In the well known procedure for solid-phase synthesis of a peptide, the desired peptide is assembled starting from an insoluble support such as benzhydryl amine or chloromethylated resin (derived from cross-linked polystyrene, and available from chemical supply houses). The amino acid at the carboxy-terminal end of the desired polypeptide, carrying protecting groups on the alpha-amino nitrogen and on any other reactive sites, is attached to the resin from solution using known peptide coupling techniques. The protecting group on the alpha-amino group is removed (leaving other protecting groups, if any, intact), and the next amino acid of the desired sequence (carrying suitable protecting groups) is attached, and so on. When the desired polypeptide has been completely built up, it is cleaved from the resin support, all protecting groups are removed, and the polypeptide is recovered. Examples of suitable protecting groups are: alpha-tert-butyloxycarbonyl for the alpha-amino-group; benzyl, 4-methoxybenzyl, or 4-methylbenzyl for the thiol group of cysteine, the beta-carboxylic acid group of aspartic acid, the gamma-carboxylic acid group of glutamic acid and the hydroxyl groups of serine, threonine, and tyrosine; benzyloxycarbonyl or a 2-chloro- or 3, 4-dimethoxy-derivative thereof for the ring nitrogens of histidine and tryptophan and the epsilon-amino group of lysine; p-nitrophenyl for the amide nitrogens of asparagine and glutamine; and nitro or tosyl for the guanidine group of arginine.

For purposes of this disclosure, accepted short-hand designations of the amino acids have been used. A complete listing is provided herein below:

| One and three-letter Amino Acid Abbreviations | | |
|---|---|---|
| A | Ala | Alanine |
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Glu | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| B | Asx | Asp or Asn, not distinguished |
| Z | Glu | Glu or Glu, not distinguished |
| X | X | Undetermined or atypical amino acid |

Among the claimed peptides 15 amino acid residues in length there are overlaps of five and ten amino acids. For example, the claimed peptides LCDLAPEAPPPTLPP and PEAPPPTLPPDMAQV have an overlap of a ten amino acid sequence, PEAPPPTLPP. In an additional example the claimed peptides VKYAGSQVASTSEVL and PSELRRIASQVKYAG have an overlap of a five amino acid sequence, VKYAG. The significance of this overlap is that a five amino acid sequence can provide the inhibitory action found in the claimed peptides 15 amino acid residues in length. Therefore, it is foreseeable from the inhibitory activity of a five amino acid sequence that a two amino acid sequence found within any of the claimed peptides would also have inhibitory activity for vWF binding.

It has been found through experimentation that the addition of at least one R and/or K residue to peptides which inhibit platelet-fibrinogen binding and platelet aggregation enhances the inhibitory activity of these peptides. In view of this ability to enhance inhibition of such binding and aggregation, peptides with R and/or K residues extending from the amino-or carboxy-termini of peptides have utility wherever it is desirable to retard or prevent the formation of a thrombus or clot in the blood (i.e. anti-thrombotic activity).

Because these different claimed peptides are from different parts of the 52/48 kDa fragment it is possible there is more than one binding site for GPIb and heparin. Therefore, linking two or more of these claimed peptides together may make a more potent inhibitor.

One or more of the peptides of the present invention can be formulated into pharmaceutical preparations for therapeutic, diagnostic, or other uses. To prepare them for intravenous administration, the compositions are dissolved in water containing physiologically compatible substances such as sodium chloride (e.g. 0.35 - 2.0 M), glycine, and the like and having a buffered pH compatible with physiological conditions. The amount to administer for the prevention of thrombosis will depend on the severity with which the patient is subject to thrombosis, but can be determined readily for any particular patient.

The following examples are given as illustrative of the present invention. The present invention is not restricted only to these examples.

Example 1

Inhibition of vWF binding to platelets

Divalent cation free platelets were prepared using Sepharose CL-2B chromatography, and used at a concentration of $10^8$/ml in an incubation volume of 125ul. $^{125}$I-vWF was added at a concentration of 5 ug/ml and the competing ligand, LCDLAPEAPPPTLPP at a concentration of 2.16 ug/ul. Ristocetin was then added at a concentration of 0.75 mg/ml. After 30 minutes incubation at room temperature without agitation, two 50 ul aliquots of the mixture were layered over two separate 300 ul cushions of 20% sucrose in a 500 ul Sarstedt microfuge tube (W. Germany), and bound ligand was separated from free ligand by 4 minutes centrifugation at 12,000 g in Beckman Microfuge B. The tube tips containing bound ligand were amputated and counted in a gamma counter. Nonspecific binding was determined in the presence of a 50-fold excess of unlabeled vWF added together with the labeled ligand and specific binding was calculated by substracting nonspecific binding from the total. The synthetic peptide LCDLAPEAPPPTLPP completely inhibited von Willebrand factor binding to platelets.

Table 1 lists specific peptides within the scope of the present invention and their inhibitory activities of vWF binding to platelets. The inhibitory activities of the peptides were determined according to the procedure set forth in Example 1. 100% is that amount of radiolabeled vWF which binds to platelets in the absence of any inhibitory peptides. Value less than 100% in Table 1 reflect the inhibitory activity of these peptides.

**Table 1**

**Peptide**

**% of vWF binding to platelets**

| Peptide | % of vWF binding to platelets |
|---|---|
| 52/48 kDa Fragment | 0 |
| PEAPPPTLPPDMAQV | 3 |
| VKYAGSQVASTSEVL | 46 |

| | |
|---|---|
| RIASQVKYAGSQVAS | 44 |
| PSELRRIASQVKYAG | 17 |
| DMMERLRISQKWVRV | 26 |
| QIFSKIDRPEASRIA | 31 |
| LYVEDISEPPLHDFY | 1 |
| VSPSSLYVEDISEPP | 18 |
| VTLNPSDPEHCQICH | 19 |
| CQICHCDVVNLTCEA | 16.5 |
| SDPEHCQICHCDVVN | 45 |
| CDVVNLTCEACQEPG | 16 |
| LTCEACQEPGGLVVP | 98 |
| CQEPGGLVVPPSDAP | 16 |
| GLVVPPSDAPVSPSS | 129 |
| PSDAPVSPSSLYVED | 4 |
| ISEPPLHDFYCSRLL | 86 |
| CSRLLDLVFLLDGSS | 90 |
| DLVFLLDGSSRLSEA | 65 |
| LDGSSRLSEAEFEVL | 74 |
| RLSEAEFEVLKAFVV | 214 |
| EFEVLKAFVVDMMEP | 13 |
| KAFVVDMMERLRISQ | 28 |
| LRISQKWVRVAVVEY | 110 |
| KWVRVAVVEYHDGSH | 133 |
| AVVEYHDGSHAYIGL | 100 |
| HDGSHAYIGLKDRKR | 11 |
| AYIGLKDRKRPSELR | 38 |
| KDRKRPSELRRIASQ | 12 |
| SQVASTSEVLKYTLF | 60 |
| TSEVLKYTLFQIFSK | 328 |
| KYTLFQIFSKIDRPE | 142 |

8

| | |
|---|---|
| IDRPEASRIALLLMA | 110 |
| ASRIALLLMASQEPQ | 12 |
| SQEPQRMSRNFVRYV | 152 |
| RMSRNFVRYVQGLKK | 24 |
| FVRYVQGLKKKKVIV | 331 |
| IPVGIGPHANLKQIR | 9 |
| GPHANLKQIRLIEKQ | 9 |
| LKQIRLIEKQAPENK | 163 |
| LIEKQAPENKAFVLS | 54 |
| APENKAFVLSSVDEL | 60 |
| AFVLSSVDELEQQRD | 54 |
| SVDELEQQRDEIVSY | 43 |
| EQQRDEIVSYLCDLA | 79 |
| EIVSYLCDLAPEAPP | 20 |
| PTLPPDMAQVTVGPG | 31 |
| DMAQVTVGPGLLGVS | 64 |
| TVGPGLLGVSTLGPK | 7 |

Example 2

Inhibition of vWF binding to heparin

Assays were performed in an incubation volume of 125 $\mu$l in plastic Eppendorf tubes. Heparin-Sepharose CL-6B (Pharmacia), $^{125}$I-vWF and bovine serum albumin (BSA) (Sigma) were added to tubes at final concentrations of 5% (v/v), 1 ug/ml and 0.1% (w/v), respectively. Then, competing ligand, KDRKRPSELRRIASQ, 0.05 M Tris-0.15 M NaCl-HCl buffer, pH 7.3 (TBS) was added to the incubation mixture at a concentration of 2.16 ug/ml. After 30 minutes incubation at room temperature with occasional agitation, two 50 ul aliquots of the mixture were layered over two separate 300 ul cushions of 20% sucrose in a 500 ul Sarstedt microfuge tube (W. Germany), and bound ligand was separated from free ligand by 4 minutes centrifugation at 12,000 g in Beckman Microfuge B. The tube tips containing bound ligand were amputated and counted in a gamma counter.

Nonspecific binding was determined in the presence of 10 mg/ml heparin Na salt (porcine intestinal mucosa, grade II, 162 USP units/mg (Sigma, St. Louis, MO) added together with the labeled ligand and heparin-Sepharose Cl-6B gel and specific binding was calculated by subtracting nonspecific binding from the total. The synthetic peptide KDRKRPSELRRIASQ inhibited von Willebrand factor binding to heparin by 75%.

Table 2 lists specific peptides within the scope of the present invention and their inhibitory activities of vWF binding to heparin. The inhibitory activities of the peptides were determined according to the procedure set forth in Example 2. 100% is that amount of radiolabeled vWF which binds to heparin in the absence of any inhibitory peptides. Values less than 100% in Table 2 reflect the inhibitory activity of these peptides.

Table 2

| Peptide | % of vWF binding to heparin |
|---|---|
| 52/48kDa Fragment | 0 |
| LIEKQAPENKAFVLS | 53.5 |
| SQEPQRMSRNFURYV | 56 |
| KYTLFQIFSKIDRPE | 54.5 |
| DMMERLRISQKWVRV | 54.5 |
| VTLNPSDPEHCQICH | 83 |
| SDPEHCQICHCDVVN | 93 |

10

| | |
|---|---:|
| CQICHCDVVNLTCEA | 92 |
| CDVVNLTCEACQEPG | 96 |
| LTCEACQEPGGLVVP | 116 |
| CQEPGGLVVPPSDAP | 91 |
| GLVVPPSDAPVSPSS | 160 |
| PSDAPVSPSSLYVED | 106 |
| VSPSSLYVEDISEPP | 91 |
| LYVEDISEPPLHDFY | 46 |
| ISEPPLHDFYCSRLL | 52 |
| CSRLLDLVFLLDGSS | 122 |
| DLVFLLDGSSRLSEA | 116 |
| LDGSSRLSEAEFEVL | 95 |
| RLSEAEFEVLKAFVV | 125 |
| EFEVLKAFVVDMMEP | 88 |
| KAFVVDMMERLRISQ | 116 |
| LRISQKWVRVAVVEY | 54 |
| KWVRVAVVEYHDGSH | 162 |
| AVVEYHDGSHAYIGL | 99 |
| HDGSHAYIGLKDRKR | 67 |
| AYIGLKDRKRPSELR | 84 |
| PSELRRIASQVKYAG | 84 |
| RIASQVKYAGSQVAS | 119 |
| VKYAGSQVASTSEVL | 103 |
| SQVASTSEVLKYTLF | 93 |
| TSEVLKYTLFQIFSK | 157 |
| QIFSKIDRPEASRIA | 94 |
| IDRPEASRIALLLMA | 81 |
| ASRIALLLMASQEPQ | 73 |
| SQEPQRMSRNFVRYV | 36 |

| | |
|---|---|
| FVRYVQGLKKKKVIV | 188 |
| IPVGIGPHANLKQIR | 24 |
| GPHANLKQIRLIEKQ | 24 |
| LKQIRLIEKQAPENK | 5 |
| APENKAFVLSSVDEL | 190 |
| AFVLSSVDELEQQRD | 113 |
| SVDELEQQRDEIVSY | 99 |
| EQQRDEIVSYLCDLA | 93 |
| EIVSYLCDLAPEAPP | 103 |
| PTLPPDMAQVTVGPG | 94 |
| DMAQVTVGPGLLGVS | 106 |
| TVGPGLLGVSTLGPK | 30 |

Example 3

Inhibition of vWF binding to collagen

Binding of vWF to collagen. vWF for these studies was radiolabeled with $^{125}$I following the technique described by Fraker and Speck, Biochem. Biophys. Res. Commun. 80:849-857 (1978) using Iodogen (Pierce Chemical, Rockford, IL). The specific activity of the preparations used in these studies varied between 2.46-8.55 x $10^{-4}$ Ci/mg (or 9.13-31.7 x $10^6$ Bq/mg). The collagen preparation used was obtained commercially (Hormon-Chemie, Munich, FRG) and consisted of an acid-insoluble, microfibrillar equine collagen, type I. The absence of non-collagenous contaminates was determined by analysis with SDS-PAGE (5% or 7.5% acrylamide with 5% cross linking) and reduction with 5% 2-mercaptoethanol. The gels were stained with Coomassie brilliant blue R. The definition of type I collagen was based on analysis by refractory calorimetry and amino acid composition (kindly performed by Dr. John McPherson, Collagen Corporation, Palo Alto, CA).

Each binding mixture contained 12.5 ul of the collagen suspension (1 mg/ml in isotonic glucose solution, pH 2.7), 5 ul of 0.4 M phosphate buffer (mono- and disodium), pH7, and 132.5 ul containing suitably diluted [125I]vWF as well as other test reagents, when necessary, all in 0.02 M Tris, 0.150 M NaCl buffer, pH 7.4. The mixture also contained 0.48% bovine serum albumin (Fraction V, Calbiochem, La Jolla, CA). Incubation was usually at room temperature (22-25°C) for 20 min, except when indicated otherwise. A 50 ul aliquot of each experimental mixture was then layered, in duplicate, over 300 ul of 20% sucrose in the above mentioned Tris buffer, containing albumin. Separation of bound from free ligand was achieved by centrifugation for 8 min at 12,000 g, a condition that resulted in pelleting of the insoluble collagent microfibrils while the soluble vWF remained on top of the sucrose layer. The tip of the microcentrifuge tube containing the collagent pellet was then amputated an the associated radioactivity was quantitated in a multi-channel gamma-scintillation spectrometer (Packard Instruments, Downer's Grove, IL). Non-saturable (non-specific) binding was determined experimentally by adding an excess amount of unlabeled vWF into the experimental mixture. Moreover, when binding isotherms were evaluated by Scatchard-type analysis, non-saturable binding was generated as a fitted parameter from the total binding isotherm using the microcomputer-assisted program Ligand.

Inhibition with 52/48 kDa fragment

The effect of tryptic fragment 52/48 kDa on the binding of intact [125I] vWF to collagen was evaluated. The experimental mixtures were prepared as described in the preceding paragraph, with the only difference that the 52/48 kDa fragment samples tested for their inhibitory activity were added, at concentrations of 1.25, 2.55 and 5 uM, immediately before addition of [125I] vWF at a concentration of 2ug/ml. The 52/48 kDa fragment inhibition of von Willebrand factor binding to collagen was greater than 90% at the above noted

12

concentrations.

**Claims**

**Claims for the following Contracting States : BE, CH, DE, FR, GB, IT, LI, LU, NL, SE**

1. A peptide which inhibits binding of von Willebrand factor to platelets, whose sequence is selected from one of the following sequences or whose sequence is a combination of any of the following sequences: LCDLAPEAPPPTLPP; PEAPPPTLPPDMAQV; VKYAGSQVASTSEVL; SDPEHCQICHCDVVN; CDVVNLTCEACQEPG; CQEPGGLVVPPSDAP; PSDAPVSPSSLYVED; RIASQVKYAGSQVAS; PSELRRIASQVKYAG; DMMERLRISQKWVRV; EFEVLKAFVVDMMER; KAFVVDMMERLRISQ; HDGSHAYIGLKDRKR; QIFSKIDRPEASRIA; LYVEDISEPPLHDFY; VSPSSLYVEDISEPP; AYIGLKDRKRPSELR; KDRKRPSELRRIASQ; ASRIALLLMASQEPQ; RMSRNFVRYVQGLKK; VTLNPSDPEHCQICH; CQICHCDVVNLTCEA; IPVGIGPHANLKQIR; GPHANLKQIRLIEKQ; SVDELEQQRDEIVSY; EIVSYLCDLAPEAPP; PTLPPDMAQVTVGPG; TVGPGLLGVSTLGPK; LIEKQAPENKAFVLS; and APENKAFVLSSVDEL.

2. A peptide as claimed in claim 1, comprising the sequence LCDLAPEAPPPTLPP.

3. A peptide as claimed in claim 1, comprising the sequence PEAPPPTLPPDMAQV.

4. A peptide as claimed in claim 1, comprising the sequence LYVEDISEPPLHDFY.

5. A peptide whose sequence is a sequential subset of at least five amino acids from any of the sequences set out in claim 1.

6. A peptide which inhibits binding of von Willebrand factor to heparin, whose sequence is selected from one of the following sequences or whose sequence is a combination of any of the following sequences: KDRKRPSELRRIASQ; LIEKQAPENKAFVLS; SQEPQRMSRNFURYV; KYTLFQIFSKIDRPE; DMMERLRISQKWVRV; LYVEDISEPPLHDFY; ISEPPLHDFYCSRLL; SQEPQRMSRNFVRYV; IPVGIGPHANLKQIR; GPHANLKQIRLIEKQ; TVGPGLLGVSTLGPK; and LKQIRLIEKQAPENK.

7. A peptide as claimed in claim 6, comprising the sequence KDRKRPSELRRIASQ.

8. A peptide whose sequence is a sequential subset of at least five amino acids from any of the sequences set out in claim 6.

9. A peptide as claimed in any one of claims 1-5, wherein a sequence as set out in claim 1 or claim 5 is repeated.

10. A peptide as claimed in any one of claims 6-8, wherein a sequence as set out in claim 6 or claim 8 is repeated.

11. A peptide as claimed in any one of claims 1-5, comprising a combination of any of the sequences set out in claim 1.

12. A peptide as claimed in any one of claims 6-8, comprising a combination of any of the sequences set out in claim 6.

13. A peptide whose sequence is that of a peptide as claimed in any of the preceding claims, with at least one further amino acid, selected from R and K, extending from its amino and/or carboxy-terminus.

14. A peptide as claimed in claim 13, wherein the at least one further amino acid is R.

15. A peptide as claimed in claim 13, wherein the at least one further amino acid is K.

16. A peptide as claimed in claim 13, wherein the at least one further amino acid includes at least one combination of the amino acids R and K.

EP 0 255 206 B1

**17.** A therapeutic composition comprising an amount of one or more peptides as claimed in any of claims 1-16, effective to inhibit binding of von Willebrand factor to platelets in a patient, in association with a pharmaceutically acceptable carrier.

**18.** A peptide as claimed in any one of claims 1-16, for use in a method of inhibiting thrombosis in a patient comprising administering to such patient an effective amount of said peptide.

**19.** Use of a polypeptide, whose amino acid sequence is that of a 52/48 KDa fragment of von Willebrand factor or a functionally equivalent homologue, the fragment comprising an animo-terminal sequence beginning with amino-terminal amino acid residue 449 Val and extending no further than amino acid residue 729 Arg, for the manufacture of a medicament for use in a method for inhibiting binding of von Willebrand factor to heparin or collagen in a patient.

**20.** A use as claimed in claim 19, wherein the method is for inhibiting thrombosis in a patient.

**21.** Use of a therapeutic composition comprising a polypeptide, whose amino acid sequence is that of a 52/48 kDa fragment of von Willebrand factor or a functionally equivalent homologue, the fragment comprising an amino-terminal sequence beginning with amino-terminal amino acid residue 449 Val and extending no further than amino acid residue 729 Arg, in association with a pharmaceutically acceptable carrier, for the manufacture of a medicament for use in a method for inhibiting binding of von Willebrand factor to heparin or collagen in a patient.

**Claims for the following Contracting States : AT, ES, GR**

**1.** A method of preparing a peptide which inhibits binding of von Willebrand factor to platelets, whose sequence is selected from one of the following sequences or whose sequence is a combination of any of the following sequences:
LCDLAPEAPPPTLPP; PEAPPPTLPPDMAQV; VKYAGSQVASTSEVL; SDPEHCQICHCDVVN; CDVVNLTCEACQEPG; CQEPGGLVVPPSDAP; PSDAPVSPSSLYVED; RIASQVKYAGSQVAS; PSELRRIASQVKYAG; DMMERLRISQKWVRV; EFEVLKAFVVDMMER; KAFVVDMMERLRISQ; HDGSHAYIGLKDRKR; QIFSKIDRPEASRIA; LYVEDISEPPLHDFY; VSPSSLYVEDISEPP; AYIGLKDRKRPSELR; KDRKRPSELRRIASQ; ASRIALLLMASQEPQ; RMSRNFVRYVQGLKK; VTLNPSDPEHCQICH; CQICHCDVVNLTCEA; IPVGIGPHANLKQIR; GPHANLKQIRLIEKQ; SVDELEQQRDEIVSY; EIVSYLCDLAPEAPP; PTLPPDMAQVTVGPG; TVGPGLLGVSTLGPK; LIEKQAPENKAFVLS; and APENKAFVLSSVDEL,
comprising forming the peptide by any available technique.

**2.** A method as claimed in claim 1, wherein the peptide includes the sequence LCDLAPEAPPPTLPP.

**3.** A method as claimed in claim 1, wherein the peptide includes the sequence PEAPPPTLPPDMAQV.

**4.** A method according to claim 1, wherein the peptide includes the sequence LYVEDISEPPLHDFY.

**5.** A method of preparing a peptide whose sequence is a sequential subset of at least five amino acids from any of the sequences set out in claim 1, comprising forming the peptide by any available technique.

**6.** A method of preparing a peptide which inhibits binding of von Willebrand factor to heparin, whose sequence is selected from one of the following sequences or whose sequence is a combination of any of the following sequences:
KDRKRPSELRRIASQ; LIEKQAPENKAFVLS; SQEPQRMSRNFURYV; KYTLFQIFSKIDRPE; DMMERLRISQKWVRV; LYVEDISEPPLHDFY; ISEPPLHDFYCSRLL; SQEPQRMSRNFVRYV; IPVGIGPHANLKQIR; GPHANLKQIRLIEKQ; TVGPGLLGVSTLGPK; and LKQIRLIEKQAPENK.
comprising forming the peptide by any available technique.

**7.** A method as claimed in claim 6, wherein the peptide includes the sequence KDRKRPSELRRIASQ.

14

**8.** A method of preparing a peptide whose sequence is a sequential subset of at least five amino acids from any of the sequences set out in claim 6, comprising forming the peptide by any available technique.

**9.** A method as claimed in any one of claims 1-5, wherein the peptide has a sequence in which a sequence as set out in claim 1 or claim 5 is repeated.

**10.** A method as claimed in any one of claims 6-8, wherein the peptide has a sequence in which a sequence as set out in claim 6 or claim 8 is repeated.

**11.** A method as claimed in any one of claims 1-5, wherein the peptide has a combination of any of the sequences set out in claim 1.

**12.** A method as claimed in any one of claims 6-8, wherein the peptide has a combination of any of the sequences set out in claim 6.

**13.** A method of preparing a peptide whose sequence is that of a peptide as claimed in any of the preceding claims, with at least one further amino acid, selected from R and K, extending from its amino and/or carboxy-terminus, comprising forming the peptide by any available technique.

**14.** A method as claimed in claim 13, wherein the at least one further amino acid is R.

**15.** A method as claimed in claim 13, wherein the at least one further amino acid is K.

**16.** A method as claimed in claim 13, wherein the at least one further amino acid includes at least one combination of the amino acids R and K.

**17.** A method of preparing a therapeutic composition comprising admixing an amount of one or more peptides, as defined in any of claims 1-16 and effective to inhibit binding of von Willebrand factor to platelets in a patient, with a pharmaceutically acceptable carrier.

**18.** A method as claimed in any one of claims 1-16, wherein the prepared peptide is for use in a method of inhibiting thrombosis in a patient comprising administering to such patient an effective amount of said peptide.

**19.** Use of a polypeptide, whose amino acid sequence is that of a 52/48 KDa fragment of von Willebrand factor or a functionally equivalent homologue, the fragment comprising an animo-terminal sequence beginning with amino-terminal amino acid residue 449 Val and extending no further than amino acid residue 729 Arg, for the manufacture of a medicament for use in a method for inhibiting binding of von Willebrand factor to heparin or collagen in a patient.

**20.** A use as claimed in claim 19, wherein the method is for inhibiting thrombosis in a patient.

**21.** Use of a therapeutic composition comprising a polypeptide, whose amino acid sequence is that of a 52/48 kDa fragment of von Willebrand factor or a functionally equivalent homologue, the fragment comprising an amino-terminal sequence beginning with amino-terminal amino acid residue 449 Val and extending no further than amino acid residue 729 Arg, in association with a pharmaceutically acceptable carrier, for the manufacture of a medicament for use in a method for inhibiting binding of von Willebrand factor to heparin or collagen in a patient.

**22.** A method of manufacturing a medicament for use in a method for inhibiting binding of von Willebrand factor to heparin or collagen in a patient, comprising using a polypeptide whose amino acid sequence is that of a 52/48kDa fragment of von Willebrand factor or a functionally equivalent homologue, the fragment comprising an amino-terminal sequence beginning with amino-terminal amino acid residue 449Val and extending no further than amino acid residue 729 Arg.

**23.** A method as claimed in claim 22, wherein the medicament is for use in inhibiting thrombosis in a patient.

EP 0 255 206 B1

**Patentansprüche**

**Patentansprüche für folgende Vertragsstaaten : BE, CH, DE, FR, GB, IT, LI, LU, NL, SE**

1. Peptid, das die Bindung des von Willebrand-Faktors an Blutplättchen inhibiert, dessen Sequenz aus einer der folgenden Sequenzen ausgewählt ist oder dessen Sequenz eine Kombination irgendwelcher der folgenden Sequenzen ist:
LCDLAPEAPPPTLPP; PEAPPPTLPPDMAQV; VKYAGSQVASTSEVL; SDPEHCQICHCDVVN; CDVVNLTCEACQEPG; CQEPGGGLVVPPSDAP; PSDAPVSPSSLYVED; RIASQVKYAGSQVAS; PSELRRI-ASQVKYAG; DMMERLRISQKWVRV; EFEVLKAFVVDMMER; KAFVVDMMERLRISQ; HDGSHAY-IGLKDRKR; QIFSKIDRPEASRIA; LYVEDISEPPLHDFY; VSPSSLYVEDISEPP; AYIGLKDRKRPSELR; KDRKRPSELRRIASQ; ASRIALLLMASQEPQ; RMSRNFVRYVQGLKK; VTLNPSDPEHCQICH; CQICHCDVVNLTCEA; IPVGIGPHANLKQIR; GPHANLKQIRLIEKQ; SVDELEQQRDEIVSY; EIVSYLCDLAPEAPP; PTLPPDMAQVTVGPG; TVGPGLLGVSTLGPK; LIEKQAPENKAFVLS; und APENKAFVLSSVDEL.

2. Peptid nach Anspruch 1 umfassend die Sequenz LCDLAPEAPPPTLPP.

3. Peptid nach Anspruch 1 umfassend die Sequenz PEAPPPTLPPDMAQV.

4. Peptid nach Anspruch 1 umfassend die Sequenz LYVEDISEPPLHDFY.

5. Peptid, dessen Sequenz eine Unterfolge von mindestens fünf Aminosäuren aus irgendeiner der in Anspruch 1 angegebenen Sequenzen ist.

6. Peptid, das die Bindung des von Willebrand-Faktors an Heparin inhibiert, dessen Sequenz aus einer der folgenden Sequenzen ausgewählt ist oder dessen Sequenz eine Kombination irgendwelcher der folgenden Sequenzen ist:
KDRKRPSELRRIASQ; LIEKQAPENKAFVLS; SQEPQRMSRNFURYV; KYTLFQIFSKIDRPE; DMMERLRISQKWVRV; LYVEDISEPPLHDFY; ISEPPLHDFYCSRLL; SQEPQRMSRNFVRYV; IPVGIGPHANLKQIR; GPHANLKQIRLIEKQ; TVGPGLLGVSTLGPK; und LKQIRLIEKQAPENK.

7. Peptid nach Anspruch 6 umfassend die Sequenz KDRKRPSELRRIASQ.

8. Peptid, dessen Sequenz eine Unterfolge von mindestens 5 Aminosäuren aus irgendeiner der in Anspruch 6 angegebenen Sequenzen ist.

9. Peptid nach irgendeinem der Ansprüche 1 - 5, worin eine der in Anspruch 1 oder Anspruch 5 angegebenen Sequenzen wiederholt ist.

10. Peptid nach irgendeinem der Ansprüche 6 - 8, worin eine der in Anspruch 6 oder Anspruch 8 angegebenen Sequenzen wiederholt ist.

11. Peptid nach irgendeinem der Ansprüche 1 - 5 umfassend eine Kombination irgendwelcher der in Anspruch 1 angegebenen Sequenzen.

12. Peptid nach irgendeinem der Ansprüche 6 - 8 umfassend eine Kombination irgendwelcher der in Anspruch 6 angegebenen Sequenzen.

13. Peptid, dessen Sequenz die eines Peptids nach irgendeinem der vorhergehenden Ansprüche ist, mit mindestens einer weiteren Aminosäure, ausgewählt aus R und K, die seinen Amino- und/oder Carboxyterminus verlängert.

14. Peptid nach Anspruch 13, worin die mindestens eine weitere Aminosäure R ist.

15. Peptid nach Anspruch 13, worin die mindestens eine weitere Aminosäure K ist.

16. Peptid nach Anspruch 13, worin die mindestens eine weitere Aminosäure mindestens eine Kombination der Aminosäuren R und K einschließt.

16

**17.** Therapeutische Zusammensetzung umfassend eine Menge von einem oder mehreren Peptiden nach irgendeinem der Ansprüche 1 - 16, die wirksam ist, um die Bindung des von Willebrand-Faktors an Blutplättchen in einem Patienten zu inhibieren, in Assoziation mit einem pharmazeutisch verträglichen Träger.

**18.** Peptid nach irgendeinem der Ansprüche 1 - 16 zur Verwendung in einem Verfahren zur Inhibierung von Thrombose in einem Patienten, welches die Verabreichung einer wirksamen Menge des Peptids an einen solchen Patienten umfaßt.

**19.** Verwendung eines Polypeptids, dessen Aminosäuresequenz die eines 52/48 kD-Fragments des von Willebrand-Faktors oder eines funktionell äquivalenten Homologs ist, wobei das Fragment eine amino-terminale Sequenz umfaßt, die mit dem aminoterminalen Aminosäurerest 449 Val beginnt und nicht weiter als bis zum Aminosäurerest 729 Arg reicht, zur Herstellung eines Medikaments zur Verwendung in einem Verfahren zur Inhibierung der Bindung des von Willebrand-Faktors an Heparin oder Collagen in einem Patienten.

**20.** Verwendung nach Anspruch 19, worin das Verfahren zur Inhibierung von Thrombose in einem Patienten ist.

**21.** Verwendung einer therapeutischen Zusammensetzung umfassend ein Polypeptid, dessen Aminosäuresequenz die eines 52/48 kD-Fragments des von Willebrand-Faktors oder eines funktionell äquivalenten Homologs ist, wobei das Fragment eine aminoterminale Sequenz umfaßt, die mit dem aminoterminalen Aminosäurerest 449 Val beginnt und nicht weiter als bis zum Aminosäurerest 729 Arg reicht, in Assoziation mit einem pharmazeutisch verträglichen Träger, zur Herstellung eines Medikaments zur Verwendung in einem Verfahren zur Inhibierung der Bindung des von Willebrand-Faktors an Heparin oder Collagen in einem Patienten.

**Patentansprüche für folgende Vertragsstaaten : AT, ES, GR**

**1.** Verfahren zur Herstellung eines Peptids, das die Bindung des von Willebrand-Faktors an Blutplättchen inhibiert, dessen Sequenz aus einer der folgenden Sequenzen ausgewählt ist oder dessen Sequenz eine Kombination irgendwelcher der folgenden Sequenzen ist:
LCDLAPEAPPPTLPP; PEAPPPTLPPDMAQV; VKYAGSQVASTSEVL; SDPEHCQICHCDVVN; CDVVNLTCEACQEPG; CQEPGGLVVPPSDAP; PSDAPVSPSSLYVED; RIASQVKYAGSQVAS; PSELRRI-ASQVKYAG; DMMERLRISQKWVRV; EFEVLKAFVVDMMER; KAFVVDMMERLRISQ; HDGSHAY-IGLKDRKR; QIFSKIDRPEASRIA; LYVEDISEPPLHDFY; VSPSSLYVEDISEPP; AYIGLKDRKRPSELR; KDRKRPSELRRIASQ; ASRIALLLMASQEPQ; RMSRNFVRYVQGLKK; VTLNPSDPEHCQICH; CQICHCDVVNLTCEA; IPVGIGPHANLKQIR; GPHANLKQIRLIEKQ; SVDELEQQRDEIVSY; EIVSYLCDLAPEAPP; PTLPPDMAQVTVGPG; TVGPGLLGVSTLGPK; LIEKQAPENKAFVLS; und APENKAFVLSSVDEL,
welches die Bildung des Peptids durch irgendein verfügbares Verfahren umfaßt.

**2.** Verfahren nach Anspruch 1, worin das Peptid die Sequenz LCDLAPEAPPPTLPP einschließt.

**3.** Verfahren nach Anspruch 1, worin das Peptid die Sequenz PEAPPPTLPPDMAQV einschließt.

**4.** Verfahren nach Anspruch 1, worin das Peptid die Sequenz LYVEDISEPPLHDFY einschließt.

**5.** Verfahren zur Herstellung eines Peptids, dessen Sequenz eine Unterfolge von mindestens fünf Aminosäuren aus irgendeiner der in Anspruch 1 angegebenen Sequenzen ist, welches die Bildung des Peptids durch irgendein verfügbares Verfahren umfaßt.

**6.** Verfahren zur Herstellung eines Peptids, das die Bindung des von Willebrand-Faktors an Heparin inhibiert, dessen Sequenz aus einer der folgenden Sequenzen ausgewählt ist oder dessen Sequenz eine Kombination irgendwelcher der folgenden Sequenzen ist:
KDRKRPSELRRIASQ; LIEKQAPENKAFVLS; SQEPQRMSRNFURYV; KYTLFQIFSKIDRPE; DMMERLRISQKWVRV; LYVEDISEPPLHDFY; ISEPPLHDFYCSRLL; SQEPQRMSRNFVRYV; IPVGIGPHANLKQIR; GPHANLKQIRLIEKQ; TVGPGLLGVSTLGPK; und LKQIRLIEKQAPENK,

welches die Bildung des Peptids durch irgendein verfügbares Verfahren umfaßt.

7. Verfahren nach Anspruch 6, worin das Peptid die Sequenz KDRKRPSELRRIASQ einschließt.

8. Verfahren zur Herstellung eines Peptids, dessen Sequenz eine Unterfolge von mindestens fünf Aminosäuren aus irgendeiner der in Anspruch 6 angegebenen Sequenzen ist, welches die Bildung des Peptids durch irgendein verfügbares Verfahren umfaßt.

9. Verfahren nach irgendeinem der Ansprüche 1 - 5, worin das Peptid eine Sequenz aufweist, in der eine der in Anspruch 1 oder Anspruch 5 angegebenen Sequenzen wiederholt ist.

10. Verfahren nach irgendeinem der Ansprüche 6 - 8, worin das Peptid eine Sequenz aufweist, in der eine der in Anspruch 6 oder Anspruch 8 angegebenen Sequenzen wiederholt ist.

11. Verfahren nach irgendeinem der Ansprüche 1 - 5, worin das Peptid eine Kombination irgendwelcher der in Anspruch 1 angegebenen Sequenzen aufweist.

12. Verfahren nach irgendeinem der Ansprüche 6 - 8, worin das Peptid eine Kombination irgendwelcher der in Anspruch 6 angegebenen Sequenzen aufweist.

13. Verfahren zur Herstellung eines Peptids, dessen Sequenz die eines Peptids nach irgendeinem der vorhergehenden Ansprüche ist, mit mindestens einer weiteren Aminosäure, ausgewählt aus R und K, die seinen Amino- und/oder Carboxyterminus verlängert, welches die Bildung des Peptids durch irgendein verfügbares Verfahren umfaßt.

14. Verfahren nach Anspruch 13, worin die mindestens eine weitere Aminosäure R ist.

15. Verfahren nach Anspruch 13, worin die mindestens eine weitere Aminosäure K ist.

16. Verfahren nach Anspruch 13, worin die mindestens eine weitere Aminosäure mindestens eine Kombination der Aminosäuren R und K einschließt.

17. Verfahren zur Herstellung einer therapeutischen Zusammensetzung, welches das Mischen einer Menge von einem oder mehreren Peptiden nach irgendeinem der Ansprüche 1 - 16 und wirksam, um die Bindung des von Willebrand-Faktors an Blutplättchen in einem Patienten zu inhibieren, mit einem pharmazeutisch verträglichen Träger umfaßt.

18. Verfahren nach irgendeinem der Ansprüche 1 - 16, worin das hergestellte Peptid zur Verwendung in einem Verfahren zur Inhibierung von Thrombose in einem Patienten ist, welches das Verabreichen einer wirksamen Menge des Peptids an einen solchen Patienten umfaßt.

19. Verwendung eines Polypeptids, dessen Aminosäuresequenz die eines 52/48 kD-Fragments des von Willebrand-Faktors oder eines funktionell äquivalenten Homologs ist, wobei das Fragment eine aminoterminale Sequenz umfaßt, die mit dem aminoterminalen Aminosäurerest 449 Val beginnt und nicht weiter als bis zum Aminosäurerest 729 Arg reicht, zur Herstellung eines Medikaments zur Verwendung in einem Verfahren zur Inhibierung der Bindung des von Willebrand-Faktors an Heparin oder Collagen in einem Patienten.

20. Verwendung nach Anspruch 19, worin das Verfahren zur Inhibierung von Thrombose in einem Patienten ist.

21. Verwendung einer therapeutischen Zusammensetzung, umfassend ein Polypeptid, dessen Aminosäuresequenz die eines 52/48 kD-Fragments des von Willebrand-Faktors oder eines funktionell äquivalenten Homologs ist, wobei das Fragment eine aminoterminale Sequenz umfaßt, die mit dem aminoterminalen Aminosäurerest 449 Val beginnt und nicht weiter als bis zum Aminosäurerest 729 Arg reicht, in Assoziation mit einem pharmazeutisch verträglichen Träger, zur Herstellung eines Medikaments zur Verwendung in einem Verfahren zur Inhibierung der Bindung des von Willebrand-Faktors an Heparin oder Collagen in einem Patienten.

EP 0 255 206 B1

**22.** Verfahren zur Herstellung eines Medikaments zur Verwendung in einem Verfahren zur Inhibierung der Bindung des von Willebrand-Faktors an Heparin oder Collagen in einem Patienten, umfassend die Verwendung eines Polypeptids, dessen Aminosäuresequenz die eines 52/48 kD-Fragments des von Willebrand-Faktors oder eines funktionell äquivalenten Homologs ist, wobei das Fragment eine aminoterminale Sequenz umfaßt, die mit dem aminoterminalen Aminosäurerest 449 Val beginnt und nicht weiter als bis zum Aminosäurerest 729 Arg reicht.

**23.** Verfahren nach Anspruch 22, worin das Medikament für die Verwendung zur Inhibierung von Thrombose in einem Patienten ist.

**Revendications**
**Revendications pour les Etats contractants suivants : BE, CH, DE, FR, GB, IT, LI, LU, NL, SE**

**1.** Peptide inhibant la fixation du facteur de von Willebrand sur les plaquettes et dont la séquence est choisie parmi les séquences suivantes, ou est une combinaison de n'importe lesquelles des séquences suivantes :
LCDLAPEAPPPTLPP ; PEAPPPTLPPDMAQV ; VKYAGSQVASTSEVL ; SDPEHCQICHCDVVN ; CDVVNLTCEACQEPG ; CQEPGGLVVPPSDAP ; PSDAPVSPSSLYVED ; RIASQVKYAGSQVAS ; PSELRRIASQVKYAG ; DMMERLRISQKWVRV ; EFEVLKAFVVDMMER ; KAFVVDMMERLRISQ ; HDGSHAYIGLKDRKR ; QIFSKIDRPEASRIA ; LYVEDISEPPLHDFY ; VSPSSLYVEDISEPP ; AYIGLKDRKRPSELR ; KDRKRPSELRRIASQ ; ASRIALLLMASQEPQ ; RMSRNFVRYVQGLKK ; VTLNPSDPEHCQICH ; CQICHCDVVNLTCEA ; IPVGIGPHANLKQIR ; GPHANLKQIRLIEKQ ; SVDELEQQRDEIVSY ; EIVSYLCDLAPEAPP ; PTLPPDMAQVTVGPG ; TVGPGLLGVSTLGPK ; LIEKQAPENKAFVLS ; et APENKAFVLSSVDEN.

**2.** Peptide selon la revendication 1, comprenant la séquence LCDLAPEAPPPTLPP.

**3.** Peptide selon la revendication 1, comprenant la séquence PEAPPPTLPPDMAQV.

**4.** Peptide selon la revendication 1, comprenant la séquence LYVEDISEPPLHDFY.

**5.** Peptide dont la séquence est un sous-groupe séquentiel d'au moins cinq aminoacides choisis parmi n'importe lesquelles des séquences énumérées à la revendication 1.

**6.** Peptide inhibant la fixation du facteur de von Willebrand sur l'héparine, et dont la séquence est choisie parmi les séquences suivantes, ou est une combinaison de n'importe lesquelles des séquences suivantes :
KDRKRPSELRRIASQ ; LIEKQAPENKAFVLS ; SQEPQRMSRNFURYV ; KYTLFQIFSKIDRPE ; DMMERLRISQKWVRV ; LYVEDISEPPLHDFY ; ISEPPLHDFYCSRLL ; SQEPQRMSRNFVRYV ; IPVGIGPHANLKQIR ; GPHANLKQIRLIEKQ ; TVGPGLLGVSTLGPK ; et LKQIRLIEKQAPENK.

**7.** Peptide selon la revendication 6, comprenant la séquence KDRKRPSELRRIASQ.

**8.** Peptide dont la séquence est un sous-groupe séquentiel d'au moins cinq aminoacides choisis parmi n'importe lesquelles des séquences énumérées à la revendication 6.

**9.** Peptide selon l'une quelconque des revendications 1-5, dans lequel une séquence telle que définie à la revendication 1 ou à la revendication 5 est répétée.

**10.** Peptide selon l'une quelconque des revendications 6-8, dans lequel une séquence telle que définie à la revendication 6 ou à la revendication 8 est répétée.

**11.** Peptide selon l'une quelconque des revendications 1-5, comprenant une combinaison de n'importe lesquelles des séquences définies à la revendication 1.

**12.** Peptide selon l'une quelconque des revendications 6-8, comprenant une combinaison de n'importe lesquelles des séquences définies à la revendication 6.

**13.** Peptide dont la séquence est celle d'un peptide selon l'une quelconque des revendications précédentes avec au moins un aminoacide supplémentaire choisi entre R et K, s'étendant depuis sa terminaison amino et/ou carboxylique.

**14.** Peptide selon la revendication 13, dans lequel l'aminoacide supplémentaire est R.

**15.** Peptide selon la revendication 13, dans lequel l'aminoacide supplémentaire est K.

**16.** Peptide selon la revendication 13, dans lequel l'aminoacide supplémentaire comporte au moins une combinaison des aminoacides R et K.

**17.** Composition thérapeutique comprenant une certaine quantité de l'un ou plusieurs des peptides selon l'une quelconque des revendications 1-16, ayant pour effet d'inhiber la fixation du facteur de von Willebrand sur les plaquettes chez un patient, en association avec un véhicule pharmaceutiquement acceptable.

**18.** Peptide selon l'une quelconque des revendications 1-16, destiné à être utilisé dans une méthode d'inhibition de la thrombose chez un patient et consistant à administrer à ce patient une quantité efficace de ce peptide.

**19.** Utilisation d'un polypeptide dont la séquence d'aminoacides est celle d'un fragment 52/48 kDa du facteur de von Willebrand ou d'un homologue fonctionnellement équivalent, ce fragment comprenant une séquence amino-terminale commençant par un reste d'aminoacide 449 Val et ne s'étendant pas au-delà d'un reste d'aminoacide 729 Arg, en vue de la fabrication d'un médicament destiné à être utilisé dans une méthode d'inhibition de la fixation du facteur de von Willebrand sur l'héparine ou le collagène chez un patient.

**20.** Utilisation selon la revendication 19, dans laquelle la méthode vise l'inhibition de la thrombose chez un patient.

**21.** Utilisation d'une composition thérapeutique comprenant un polypeptide dont la séquence d'aminoacides est celle d'un fragment 52/48 kDa du facteur de von Willebrand ou d'un homologue fonctionnellement équivalent, ce fragment comprenant une séquence amino-terminale commençant par un reste d'aminoacide 449 Val et ne s'étendant pas au-delà d'un reste d'aminoacide 729 Arg, en association avec un véhicule pharmaceutiquement acceptable en vue de la fabrication d'un médicament destiné à être utilisé dans une méthode d'inhibition de la fixation du facteur de von Willebrand sur l'héparine ou le collagène chez un patient.

**Revendications pour les Etats contractants suivants : AT, ES, GR**

**1.** Procédé pour la préparation d'un peptide inhibant la fixation du facteur de von Willebrand sur les plaquettes, et dont la séquence est choisie parmi les séquences suivantes, ou est une combinaison de n'importe lesquelles des séquences suivantes :
LCDLAPEAPPPTLPP ; PEAPPPTLPPDMAQV ; VKYAGSQVASTSEVL ; SDPEHCQICHCDVVN ; CDVVNLTCEACQEPG ; CQEPGGLVVPPSDAP ; PSDAPVSPSSLYVED ; RIASQVKYAGSQVAS ; PSELRRIASQVKYAG ; DMMERLRISQKWVRV ; EFEVLKAFVVDMMER ; KAFVVDMMERLRISQ ; HDGSHAYIGLKDRKR ; QIFSKIDRPEASRIA ; LYVEDISEPPLHDFY ; VSPSSLYVEDISEPP ; AYIGLKDRKRPSELR ; KDRKRPSELRRIASQ ; ASRIALLLMASQEPQ ; RMSRNFVRYVQGLKK ; VTLNPSDPEHCQICH ; CQICHCDVVNLTCEA ; IPVGIGPHANLKQIR ; GPHANLKQIRLIEKQ ; SVDE-LEQQRDEIVSY ; EIVSYLCDLAPEAPP ; PTLPPDMAQVTVGPG ; TVGPGLLGVSTLGPK ; LIEKQAPEN-KAFVLS ; et APENKAFVLSSVDEN
et consistant à former le peptide par toute technique disponible.

**2.** Procédé selon la revendication 1, dans lequel le peptide comporte la séquence LCDLAPEAPPPTLPP.

**3.** Procédé selon la revendication 1, dans lequel le peptide comporte la séquence PEAPPPTLPPDMAQV.

**4.** Procédé selon la revendication 1, dans lequel le peptide comporte la séquence LYVEDISEPPLHDFY.

5. Procédé pour la préparation d'un peptide dont la séquence est un sous-groupe séquentiel d'au moins cinq aminoacides choisis parmi les séquences définies à la revendication 1, consistant à former le peptide par toute technique disponible.

6. Procédé pour la préparation d'un peptide inhibant la fixation du facteur de von Willebrand sur l'héparine, et dont la séquence est choisie parmi les séquences suivantes, ou est une combinaison de n'importe lesquelles des séquences suivantes :
KDRKRPSELRRIASQ ; LIEKQAPENKAFVLS ; SQEPQRMSRNFURYV ; KYTLFQIFSKIDRPE ; DMMERL-RISQKWVRV ; LYVEDISEPPLHDFY ; ISEPPLHDFYCSRLL ; SQEPQRMSRNFVRYV ; IPVGIGPHANLK-QIR ; GPHANLKQIRLIEKQ ; TVGPGLLGVSTLGPK ; et LKQIRLIEKQAPENK
et consistant à former le peptide par toute technique disponible.

7. Procédé selon la revendication 6, dans lequel le peptide comporte la séquence KDRKRPSELRRIASQ.

8. Procédé pour la préparation d'un peptide dont la séquence est un sous-groupe séquentiel d'au moins cinq aminoacides choisis parmi les séquences définies à la revendication 6, consistant à former le peptide par toute technique disponible.

9. Procédé selon l'une quelconque des revendications 1-5, dans lequel le peptide a une séquence dans laquelle une séquence telle que définie à la revendication 1 ou à la revendication 5 est répétée.

10. Procédé selon l'une quelconque des revendications 6-8, dans lequel le peptide a une séquence dans laquelle une séquence telle que définie dans la revendication 6 ou dans la revendication 8 est répétée.

11. Procédé selon l'une quelconque des revendications 1-5, dans lequel le peptide comporte une combinaison de n'importe lesquelles des séquences définies à la revendication 1.

12. Procédé selon l'une quelconque des revendications 6-8, dans lequel le peptide comporte une combinaison de n'importe lesquelles des séquences définies à la revendication 6.

13. Procédé pour la préparation d'un peptide dont la séquence est celle d'un peptide selon l'une quelconque des revendications précédentes, avec au moins un aminoacide supplémentaire choisi entre R et K, s'étendant depuis sa terminaison amino et/ou carboxylique, et consistant à former le peptide par toute technique disponible.

14. Procédé selon la revendication 13, dans lequel l'aminoacide supplémentaire est R.

15. Procédé selon la revendication 13, dans lequel l'aminoacide supplémentaire est K.

16. Procédé selon la revendication 13, dans lequel l'aminoacide supplémentaire comporte au moins une combinaison des aminoacides R et K.

17. Procédé pour la préparation d'une composition thérapeutique consistant à mélanger une certaine quantité d'un ou plusieurs peptides tels que définis dans l'une quelconque des revendications 1-16 et ayant pour effet d'inhiber la fixation du facteur de von Willebrand sur les plaquettes chez un patient, avec un véhicule pharmaceutiquement acceptable.

18. Procédé selon l'une quelconque des revendications 1-16, dans lequel le peptide préparé est destiné à être utilisé dans une méthode d'inhibition de la thrombose chez un patient et consistant à administrer à ce patient une quantité efficace de ce peptide.

19. Utilisation d'un polypeptide dont la séquence d'aminoacides est celle d'un fragment 52/48 kDa du facteur de von Willebrand ou d'un homologue fonctionnellement équivalent, ce fragment comprenant une séquence amino-terminale commençant par un reste d'aminoacide 449 Val et ne s'étendant pas au-delà d'un reste d'aminoacide 729 Arg, en vue de la fabrication d'un médicament destiné à être utilisé dans une méthode d'inhibition de la fixation du facteur de von Willebrand sur l'héparine ou le collagène chez un patient.

**20.** Utilisation selon la revendication 19, dans laquelle la méthode vise l'inhibition de la thrombose chez un patient.

**21.** Utilisation d'une composition thérapeutique comprenant un polypeptide dont la séquence d'aminoacides est celle d'un fragment 52/48 kDa du facteur de von Willebrand ou d'un homologue fonctionnellement équivalent, ce fragment comprenant une séquence amino-terminale commençant par un reste d'aminoacide 449 Val et ne s'étendant pas au-delà d'un reste d'aminoacide 729 Arg, en association avec un véhicule pharmaceutiquement acceptable en vue de la fabrication d'un médicament destiné à être utilisé dans une méthode d'inhibition de la fixation du facteur de von Willebrand sur l'héparine ou le collagène sur un patient.

**22.** Procédé pour la fabrication d'un médicament destiné à être utilisé dans une méthode d'inhibition de la fixation du facteur de von Willebrand sur l'héparine ou le collagène chez un patient, consistant à utiliser un polypeptide dont la séquence d'aminoacides est celle d'un fragment 52/48 kDa du facteur de von Willebrand ou un homologue fonctionnellement équivalent, ce fragment comportant une séquence amino-terminale commençant par un reste d'aminoacide 449 Val et ne s'étendant pas au-delà du reste d'aminoacide 729 Arg.

**23.** Procédé selon la revendication 22, dans lequel le médicament est destiné à être utilisé pour inhiber la thrombose chez un patient.